(19) Europäisches Patentamt
European Patent Office
Office européen des brevets

(11) Veröffentlichungsnummer : **0 465 405 A2**

(12) # EUROPÄISCHE PATENTANMELDUNG

(21) Anmeldenummer : **91810406.8**

(22) Anmeldetag : **29.05.91**

(51) Int. Cl.[5] : **C08K 5/3415,** C08K 5/37, C08K 5/41, C08L 57/08

(30) Priorität : **07.06.90 CH 1907/90**

(43) Veröffentlichungstag der Anmeldung : **08.01.92 Patentblatt 92/02**

(84) Benannte Vertragsstaaten : **BE DE FR GB IT**

(71) Anmelder : **CIBA-GEIGY AG**
**Klybeckstrasse 141**
**CH-4002 Basel (CH)**

(72) Erfinder : **Drewes, Rolf, Dr.**
**Am Buchacker 1**
**W-6145 Lindenfels (DE)**
Erfinder : **Friedrich, Hans-Helmut**
**Am Rauhenstein 8**
**W-6147 Lautertal 2 (DE)**

(54) **Substituierte Pyrrole als Stabilisatoren für chlorhaltige Polymerisate.**

(57) Zusammensetzung enthaltend
a) ein chlorhaltiges Polymerisat und
b) mindestens eine Verbindung der Formel I,

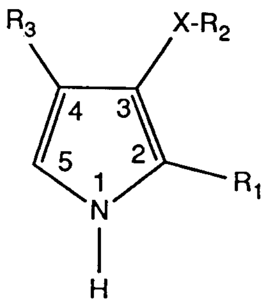

(I)

worin X eine Gruppe

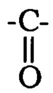 , 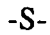 , 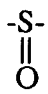

oder

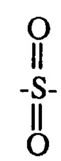

bedeutet, $R_1$ und $R_2$ unabhängig voneinander $C_1$-$C_{20}$-Alkyl, durch Hydroxy und/oder Halogen subsrituiertes $C_1$-$C_{10}$-Alkyl, $C_3$-$C_{20}$-Alkenyl, $C_5$-$C_{12}$-Cycloalkyl, durch $C_1$-$C_4$-Alkyl substituiertes $C_5$-$C_8$-Cycloalkyl, Phenyl oder durch 1 bis 3 Reste $A_1$ substituiertes Phenyl sind, wobei die Reste $A_1$ unabhängig voneinander $C_1$-$C_{10}$-Alkyl, Halogen, Hydroxy, Methoxy oder Ethoxy bedeuten,
$R_1$ und $R_2$ ferner $C_7$-$C_{10}$-Phenylalkyl oder am Phenyl durch 1 bis 3 Reste $A_2$ substituiertes $C_7$-$C_{10}$-Phenylalkyl sind, wobei die Reste $A_2$ unabhängig voneinander $C_1$-$C_{20}$ Alkyl, Halogen, Hydroxy, Methoxy oder Ethoxy bedeuten, und
$R_3$ Phenyl oder durch 1 bis 3 Reste $A_3$ substituiertes Phenyl ist, wobei die Reste $A_3$ unabhängig voneinander $C_1$-$C_{10}$-Alkyl, Halogen, Hydroxy, Methoxy, Ethoxy oder ($C_1$-$C_8$-Alkyl)oxycarbonyl sind, mit den Bedingungen, dass mindestens einer der Reste $R_1$ und $R_2$ Phenyl oder definitionsgemäss substituiertes Phenyl ist und $R_1$ verschieden von Methyl ist, wenn $R_2$ Phenyl bedeutet.

EP 0 465 405 A2

Jouve, 18, rue Saint-Denis, 75001 PARIS

Einige Verbindungen der Formel I sind neu.

Die vorliegende Erfindung betrifft chlorhaltige Polymerisate enthaltend substituierte Pyrrole, die Verwendung der Pyrrolverbindungen zum Stabilisieren von chlorhaltigen Polymerisaten gegen thermischen und lichtinduzierten Abbau sowie neue Pyrrole.

Es ist bekannt, dass chlorhaltige Polymerisate gegen den schädigenden Einfluss von Licht und Wärme, insbesondere bei der Verarbeitung zu Formteilen, geschützt werden müssen. Die Verwendung von Pyrrolen als Stabilisatoren für chlorhaltige Thermoplasten wird z.B. in US-A-4,369,276 und GB-A-2078761 beschrieben.

Die Herstellung von substituierten Pyrrolen wird beispielsweise in folgenden Publikationen beschrieben: L.G. Tikhonova et al.; Zhurnal Organicheskoi Khimii 11, 2510- 14 (1975), H.A. Houwing et al.; Tetrahedron Letters 2, 143-6 (1976), P.F. dos Santos Filho et al.; Angew. Chem. 89, 672-3 (1977) und H.A. Houwing et al.; J. Heterocycl. Chem. 18, 1127-32 (1981).

Die Verwendung von Pyrrolen als Arzneimittel ist z.B. aus BE-A-715 405 bekannt.

Die vorliegende Erfindung betrifft Zusammensetzungen enthaltend

a) ein chlorhaltiges Polymerisat und

b) mindestens eine Verbindung der Formel I,

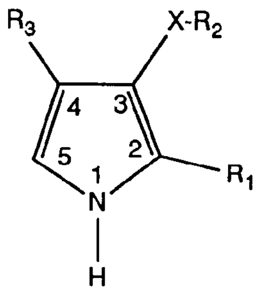

(I)

worin X eine Gruppe

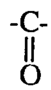 , -S- , 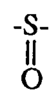

oder

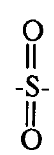

bedeutet, $R_1$ und $R_2$ unabhängig voneinander $C_1$-$C_{20}$-Alkyl, durch Hydroxy und/oder Halogen substituiertes $C_1$-$C_{10}$-Alkyl, $C_3$-$C_{20}$-Alkenyl, $C_5$-$C_{12}$-Cycloalkyl, durch $C_1$-$C_4$-Alkyl substituiertes $C_5$-$C_8$-Cycloalkyl, Phenyl oder durch 1 bis 3 Reste $A_1$ substituiertes Phenyl sind, wobei die Reste $A_1$ unabhängig voneinander $C_1$-$C_{10}$-Alkyl, Halogen, Hydroxy, Methoxy oder Ethoxy bedeuten,

$R_1$ und $R_2$ ferner $C_7$-$C_{10}$-Phenylalkyl oder am Phenyl durch 1 bis 3 Reste $A_2$ substituiertes $C_7$-$C_{10}$-Phenylalkyl sind, wobei die Reste $A_2$ unabhängig voneinander $C_1$-$C_{20}$-Alkyl, Halogen, Hydroxy, Methoxy oder Ethoxy bedeuten, und

$R_3$ Phenyl oder durch 1 bis 3 Reste $A_3$ substituiertes Phenyl ist, wobei die Reste $A_3$ unabhängig voneinander $C_1$-$C_{10}$-Alkyl, Halogen, Hydroxy, Methoxy, Ethoxy oder ($C_1$-$C_8$-Alkyl)oxycarbonyl sind, mit den Bedingungen, dass mindestens einer der Reste $R_1$ und $R_2$ Phenyl oder definitionsgemäss substituiertes Phenyl ist und $R_1$ verschieden von Methyl ist, wenn R2 Phenyl bedeutet.

Die Verbindungen der Formel (I) zeichnen sich durch eine sehr gute stabilisierende Wirkung sowohl gegen thermischen als auch lichtinduzierten Abbau aus. Besonders bemerkenswert ist die langzeitstabilisierende Wirkung gegen thermischen Abbau.

Vorzugsweise ist $R_1$ verschieden von $C_1$-$C_3$-Alkyl, insbesondere $C_1$ -$C_{20}$-Alkyl, wenn $R_2$ Phenyl bedeutet. Halogen bedeutet bevorzugt Chlor.

Alkyl mit bis zu 20 C-Atomen bedeutet zum Beispiel Methyl, Ethyl, Propyl, Butyl, t-Butyl, Pentyl, Hexyl, Heptyl, Octyl, i-Octyl, i-Nonyl, Decyl, Dodecyl oder Octadecyl.

Durch Hydroxy und/oder Halogen substituiertes $C_1$-$C_{10}$-Alkyl bedeutet zum Beispiel 5-Hydroxypenty1, 2,3,5-Trihydroxypenty1 oder 5-Chlorpentyl.

$C_3$-$C_{20}$-Alkenyl ist beispielsweise Allyl, 2-Methallyl, 3-Methylbut-2-enyl, 3-Methylbut-3-enyl, Hexenyl, Decenyl, Undecenyl, Heptadecenyl oder Oleyl. Bevorzugte Bedeutungen sind Allyl, Methallyl und Oleyl.

$C_5$-$C_{12}$-Cycloalkyl bedeutet zum Beispiel Cyclopentyl, Cyclohexyl, Cycloheptyl, Cyclooctyl, Cyclodecyl oder Cyclododecyl. $C_5$-$C_7$-Cycloalkyl, insbesondere Cyclohexyl, ist bevorzugt.

$C_5$-$C_8$-Cycloalkyl, welches durch $C_1$-$C_4$-Alkyl, insbesondere Methyl substituiert ist, bedeutet zum Beispiel Methylcyclohexyl oder tert-Butylcyclohexyl.

Beispiele für Phenyl, welches durch 1 bis 3 definitionsgemässe Reste substituiert ist, sind o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 2-Methyl-4-tert-butylphenyl, 2-Ethylphenyl, 2,6-Diethylphenyl, 2,6-Diethyl-4-methylphenyl, 2,6-Diisopropylphenyl, 4-tert-Butylphenyl, p-Nonylphenyl, o-, m- oder p-Chlorphenyl, 2,3-Dichlorphenyl, 2,4-Dichlorphenyl, 2,5-Dichlorphenyl, 2,6-Dichlorphenyl, 3,4-Dichlorphenyl, 2,4,5-Trichlorphenyl, 2,4,6-Trichlorphenyl, o-, m- oder p-Hydroxyphenyl, o-, m- oder p-Methoxyphenyl, o- oder p-Ethoxyphenyl, 2,4-Dimethoxyphenyl, 2,5-Dimethoxyphenyl, 2,5-Diethoxyphenyl, o-, m- oder p-Methoxycarbonyl, 2-Chlor-6-methylphenyl, 3-Chlor-2-methylphenyl, 3-Chlor-4-methylphenyl, 4-Chlor-2-methylphenyl, 5-Chlor-2-methylphenyl, 2,6-Dichlor-3-methylphenyl, 2-Hydroxy-4-methylphenyl, 3-Hydroxy-4-methylphenyl, 2-Methoxy-5-methylphenyl, 4-Methoxy-2-methylphenyl, 3-Chlor-4-methoxyphenyl, 3-Chlor-6-methoxyphenyl, 3-Chlor-4,6-dimethoxyphenyl und 4-Chlor-2,5-dimethoxyphenyl.

$C_7$-$C_{10}$-Phenylalkyl ist zum Beispiel Benzyl oder 2-Phenylethyl. Benzyl ist bevorzugt. Falls die Phenylgruppe in diesen Resten durch 1 bis 3 definitionsgemässe Gruppen substituiert ist, kann sie die oben angegebenen Bedeutungen annehmen. An der Phenylgruppe durch $C_1$-$C_{20}$-Alkyl, bevorzugt $C_8$-$C_{14}$-Alkyl, substituiertes $C_7$-$C_{10}$-Phenylalkyl ist eine der bevorzugten Bedeutungen. Als Beispiel ist ferner Dodecylbenzyl zu nennen.

Von Interesse sind Zusammensetzungen, worin $R_1$ und $R_2$ unabhängig voneinander $C_1$-$C_{20}$-Alkyl, durch Hydroxy substituiertes $C_1$-$C_5$-Alkyl, $C_3$-$C_{17}$-Alkenyl, $C_5$-$C_7$-Cycloalkyl, durch $C_1$-$C_4$-Alkyl substituiertes $C_5$-$C_7$-Cycloalkyl, Phenyl, durch 1 bis 3 Reste A1 substituiertes Phenyl, $C_7$-$C_{10}$-Phenylalkyl oder am Phenyl durch 1 bis 3 Reste $A_2$ substituiertes $C_7$-$C_{10}$-Phenylalkyl bedeuten und $R_3$ Phenyl oder durch 1 bis 3 Reste $A_3$ substituiertes Phenyl bedeutet.

Bevorzugt sind auch Zusammensetzungen, worin $R_1$ und $R_2$ unabhägig voneinänder $C_1$-$C_{17}$-Alkyl, durch Hydroxy substituiertes $C_1$-$C_5$-Alkyl, $C_3$-$C_{17}$-Alkenyl, Cyclohexyl, durch $C_1$-$C_4$-Alkyl substituiertes Cyclohexyl, Phenyl oder durch 1 bis 3 Reste $A_1$ substituiertes Phenyl sind, wobei die Reste $A_1$ unabhängig voneinander $C_1$-$C_4$-Alkyl, Chlor, Hydroxy, Methoxy oder Ethoxy bedeuten, $R_1$ und $R_2$ ferner Benzyl oder am Phenyl durch 1 bis 3 Reste $A_2$ substituiertes Benzyl sind, wobei die Reste $A_2$ unabhängig voneinander $C_8$-$C_{14}$-Alkyl, Chlor, Hydroxy, Methoxy oder Ethoxy bedeuten, und $R_3$ Phenyl oder durch 1 bis 3 Reste $A_3$ substituiertes Phenyl ist, wobei die Reste $A_3$ unabhängig voneinander $C_1$-$C_4$-Alkyl, Chlor, Hydroxy, Methoxy oder Ethoxy sind.

Ebenfalls bevorzugt sind Zusammensetzungen, worin $R_1$ und $R_2$ unabhängig voneinander $C_1$-$C_{17}$-Alkyl, durch Hydroxy substituiertes $C_1$-$C_5$-Alkyl oder Phenyl bedeuten und $R_3$ phenyl oder durch einen Rest $A_3$ substituiertes Phenyl ist, wobei $A_3$ $C_1$-$C_4$-Alkyl, Chlor oder Methoxy bedeutet.

Der Rest $R_1$ bedeutet vorzugsweise Phenyl.

Besonders bevorzugt sind Zusammensetzungen, worin $R_1$ und $R_3$ Phenyl bedeuten und $R_2$ Phenyl oder $C_4$-$C_{17}$-Alkyl ist.

Der Rest X bedeutet bevorzugt eine Gruppe

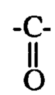

oder -S-, insbesondere

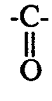 .

Bevorzugte Beispiele für Verbindungen der Formel I sind 2,4-Diphenyl-3-benzoylpyrrol und 2,4-Diphenyl-

3-octadecanoylpyrrol.

Bei den chlorhaltigen Polymerisaten handelt es sich bevorzugt um Vinylchloridhomopolymere oder -copolymere. Als Comonomere für die Copolymerisate kommen z.B. in Frage: Vinylacetat, Vinylidenchlorid, Transdichlorethen, Ethylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA, ABS und MBS. Bevorzugte Substrate sind auch Mischungen der vorstehend genannten Homo- und Copolymerisate, insbesondere Vinylchlorid-Homopolymerisate, mit anderen thermoplastischen oder/und elastomeren Polymeren, insbesondere mit ABS, MBS, NBR, SAN, EVA.

Weiterhin bevorzugt sind Suspensions- und Massepolymere sowie Emulsionspolymere.

Als chlorhaltiges Polymerisat ist Polyvinylchlorid besonders bevorzugt.

Es ist möglich, die Verbindungen der Formel I allein oder zusammen mit bekannten Thermostabilisatoren einzusetzen, wie z.B. Organozinnverbindungen, Bleiverbindungen, organischen Antimonverbindungen, Me(II)-Phenolaten, insbesondere $C_7$-$C_2$-Alkylphenolaten, beispielsweise Nonylphenolat, oder Me(II)-Carboxylaten. Me(II) bedeutet z.B. Ba, Ca, Mg, Cd oder Zn. Bei den Carboxylaten handelt es sich bevorzugt um Salze von Carbonsäuren mit 7 bis 20 C-Atomen, beispielsweise Benzoate, Alkenoate oder Alkanoate, bevorzugt Stearate, Oleate, Laurate, Palmitate, Hydroxystearate oder 2-Ethylhexanoate. Besonders bevorzugt sind Stearate, Oleate und p-tert-Butylbenzoate. Beispiele für Organozinnverbindungen, Bleiverbindungen und organische Antimonverbindungen sind die in US-A-4 743 640 Spalte 3, Zeile 48 bis Spalte 5, Zeile 38 genannten Verbindungen.

Zusätzlich können die mit den Verbindungen der Formel I stabilisierten chlorhaltigen Polymerisate in üblichen Mengen herkömmliche PVC-Stabilisatoren enthalten, wie beispielsweise Phosphite oder Epoxyverbindungen.

Bei den Phosphiten handelt es sich bevorzugt um solche der Formeln

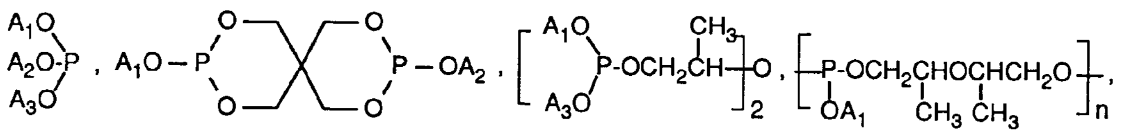

worin $A_1$, $A_2$ und $A_3$ unabhängig voneinander $C_4$-$C_{18}$-Alkyl, $C_6$-$C_{18}$-Alkenyl, $C_5$-$C_7$-Cycloalkyl, Phenyl oder durch ein bis drei $C_1$-$C_{12}$-Alkylgruppen substituiertes Phenyl bedeuten.

Beispiele sind Trioctyl-, Tridecyl-, Tridodecyl-, Triteaadecyl-, Tristearyl-, Trioleyl-, Triphenyl-, Trikresyl-, Tris-p-nonylphenyl- und Tricyclohexylphosphit. Bevorzugt sind die Aryldialkyl- sowie die Alkyldiaryl-phosphite, wie z.B. Phenyldidecyl-, (2,4-Di-tert-butylphenyl)didodecyl-, (2,6-Di-tert-butylphenyl)didodecyl-phosphit und die Dialkyl- und Diaryl-pentaerythyrit-diphosphite, wie z.B. Distearylpentaerythrit-diphosphit. Ebenfalls bevorzugt sind die Tetraphenyl- und Tetralkyl-[dipropylenglykol- 1,2]-diphosphite und die Poly-[dipropylenglykol- 1,2-phenylphosphite] sowie die Poly-[dipropylenglykol- 1,2-alkylphosphite].

Besonders bevorzugte organische Phosphite sind Distearyl-pentaerythritdiphosphit, Tris(nonylphenyl)phosphit, Phenyldidecylphosphit, Teaaphenyl-[dipropylenglykol- 1,2]-diphosphit und Poly- [dipropylenglykol- 1,2-phenylphosphit].

Bei der Epoxyverbindung handelt es sich bevorzugt um epoxidierte Oele und epoxidierte Fettsäureester, z.B. epoxidiertes Sojabohnenöl, epoxidiertes Butyloleat und epoxidiertes Octyloleat.

Ein bevorzugter Gegenstand der Erfindung sind daher auch Zusammensetzungen enthaltend ausser der Komponente a) und einer Verbindung der Formel I mindestens ein Me(II)-Carboxylat und/oder Me(II)-Phenolat, wobei Me(II) Ba, Ca, Mg, Cd oder Zn bedeutet.

Gemäss einer weiteren Bevorzugung enthalten die erfindungsgemässen Zusammensetzungen ausser der Komponente a) und einer Verbindung der Formel I mindestens ein Me(II)-Carboxylat, wobei Me(II) Ba, Ca, Mg oder Zn bedeutet. Gemische aus Ba/Zn- oder Ca/Zn-Carboxylaten werden dabei als Costabilisatoren besonders bevorzugt.

Ebenfalls bevorzugt sind Zusammensetzungen enthaltend ausser der Komponente a) und einer Verbindung der Formel I eine Epoxyverbindung und/oder ein Phosphit und gegebenenfalls ein Me(II)-Carboxylat und-/oder Me(II)-Phenolat.

Die bekannten Thermostabilisatoren (z.B. Carboxylate) können in dem zu stabilisierenden Material in einer dem Fachmann bekannten Konzentration vorliegen, wie zum Beispiel in Mengen von 0,05 bis 5 Gew.%.

Die Phosphite werden z.B. in Konzenaationen von 0,3 bis 5, vorzugsweise 0,5 bis 1 Gew.% und die Epoxyverbindungen, wie z.B. das epoxidierte Sojabohnenöl, zweckmässig in Konzenaationen von I bis 8, vorzugsweise I bis 3 Gew.% eingesetzt.

Die Verbindungen der Formel I werden beispielsweise in Mengen von 0,05 bis 5, bevorzugt 0,05 bis 3, insbesondere 0,1 bis 2,0 Gew.% in das chlorhaltige Polymerisat eingearbeitet.

Die Angabe Gew.% bezieht sich jeweils auf das zu stabilisierende Material.

Je nach dem Verwendungszweck der Polymerisate können vor oder bei der Einarbeitung der Stabilisatoren auch weitere Zusätze eingearbeitet werden, wie zum Beispiel phenolische Antioxidantien, Gleitmittel (bevorzugt Montanwachse oder Glycerinester), Fettsäweester, Paraffine, Weichmacher, Füllstoffe, Russ, Asbest, Kaolin, Talk, Glasfasem, Modifikatoren (wie etwa Schlagzäh-Zusätze), optische Aufheller, Pigmente, Lichtschutzmittel, UV-Absorber, Flammschutzmittel oder Antistatika.

Weitere mögliche Zusätze sind ferner β-Aminocrotonate, z.B. die in DE-A-804 442, DE-A-807 207 und JP-A-75/17454 beschriebenen Verbindungen, Pyrrole, z.B. die in EP-A-22 087 angegebenen Verbindungen, Aminouracile, z.B. die in EP-A-65 934 offenbarten Verbindungen, Aminothiouracile, z.B. die aus EP-A-41 479 bekannten Verbindungen, Polyole, z.B. die in DE-A-3 019 910 beschriebenen Verbindungen, β-Diketone, z.B. die in DE-A-2 600 516 angegebenen Verbindungen, oder auch Gemische aus β-Diketonen und Hydrotalciten, wie z.B. in EP-A-63 180 beschrieben.

Ebenfalls bevorzugt sind Zusaminensetzungen enthaltend ausser der Komponente a) und einer Verbindung der Formel 1, ein β-Diketon, wie z.B. Phenyleicosan-1,3-dion, und gegebenenfalls ein Me(II)-Carboxylat und/oder Me(II)-Phenolat.

Die Einarbeitung der Stabilisatorkomponenten in das chlorhaltige Polymerisat erfolgt am günstigsten, wie üblich, auf einem Mischwalzwerk, z.B. einem 2-Walzenstuhl bei Temperaturen zwischen 150° und 200°C. Im allgemeinen lässt sich eine genügende Homogenisierung innerhalb von 5 bis 15 Minuten erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachem gearbeitet.

Die erfindungsgemässen Zusammensetzungen können nach den dafür gebräuchlichen Formgebungsverfahren, z.B. durch Exausion, Spritzgiessen oder kalandrieren zu Formteilen verarbeitet werden. Auch die Verwendung als Plastisole ist möglich.

Bevorzugt werden die erfindungsgemässen Zusammensetzungen für die Herstellung von Elekaokabeln, Hohlkörpem, z.B. Roben, und insbesondere Folien in der Kraftfahrzeugindustrie verwendet. Diese Verwendung ist ebenfalls Gegenstand der Erfindung. Ein besonders bevorzugtes Einsatzgebiet ist die Herstellung von Folien für kraftfahrzeuginnenräume, insbesondere wie sie in der DE-A 3 227 107 und der DE-A 3 401 482 beschrieben sind.

Die erfindungsgemässen Zusammensetzungen werden besonders vorteilhaft zur Herstellung von Tiefzieh- und Weichfolien auf PVC-Basis herangezogen, vor allem zur Verwendung in der kraftfahrzeugindustrie.

Ein weiterer Gegenstand der Erfindung ist die Verwendung der Verbindungen der Formel I zum Stabilisieren von chlorhaltigen Polymerisaten gegen thermischen und lichtinduzierten Abbau.

Ebenfalls Gegenstand der Erfindung sind die neuen Verbindungen der Formel Ib,

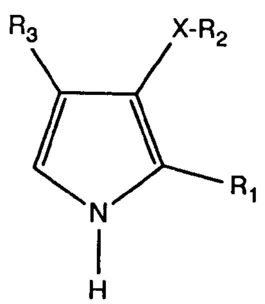

(Ib)

worin X eine Gruppe

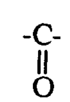 , -S- , 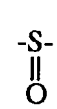

oder

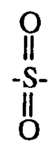

bedeutet, $R_1$ und $R_2$ unabhängig voneinander $C_1$-$C_{20}$-Alkyl, durch Halogen substituiertes $C_1$-$C_{10}$-Alkyl, $C_3$-$C_{20}$-Alkenyl, $C_5$-$C_{12}$-Cycloalkyl, durch $C_1$-$C_4$-Alkyl substituiertes $C_5$-$C_8$-Cycloalkyl, Phenyl oder durch 1 bis 3 Reste $A_1$ substituiertes Phenyl sind, wobei die Reste $A_1$ unabhängig voneinander $C_1$-$C_{10}$-Alkyl, Halogen, Hydroxy, Methoxy oder Ethoxy bedeuten,
$R_1$ und $R_2$ ferner $C_7$-$C_{10}$-Phenylalkyl oder am Phenyl durch 1 bis 3 Reste $A_2$ substituiertes $C_7$-$C_{10}$-Phenylalkyl sind, wobei die Reste $A_2$ unabhängig voneinander $C_1$-$C_{20}$-Alkyl, Halogen, Hydroxy, Methoxy oder Ethoxy bedeuten, und $R_3$ Phenyl oder durch 1 bis 3 Reste $A_3$ substituiertes Phenyl bedeutet, wobei die Reste $A_3$ unabhängig voneinander $C_1$-$C_{10}$-Alkyl, Halogen, Hydroxy, Methoxy, Ethoxy oder ($C_1$-$C_8$-Alkyl)oxycarbonyl sind, mit den Bedingungen, dass mindestens einer der Reste $R_1$ und $R_2$ Phenyl oder definitionsgemäss substituiertes Phenyl ist und $R_1$ verschieden von Methyl und Phenyl ist, wenn $R_2$ Phenyl bedeutet.

Bevorzugte Bedeutungen für die Variablen X, $R_1$, $R_2$ und $R_3$ sind solche, wie sie oben für die Formel I angegeben sind.

Die Verbindungen der Formel I können in Analogie zu bekannten Verfahren, beispielsweise der Knorr'schen Pyrrolsynthese oder wie von P.F. dos Santos Filho et al. in Angew Chem. 89, 672 ( 1977) beschrieben, gemäss folgendem Reaktionsschema hergestellt werden.

Schema A

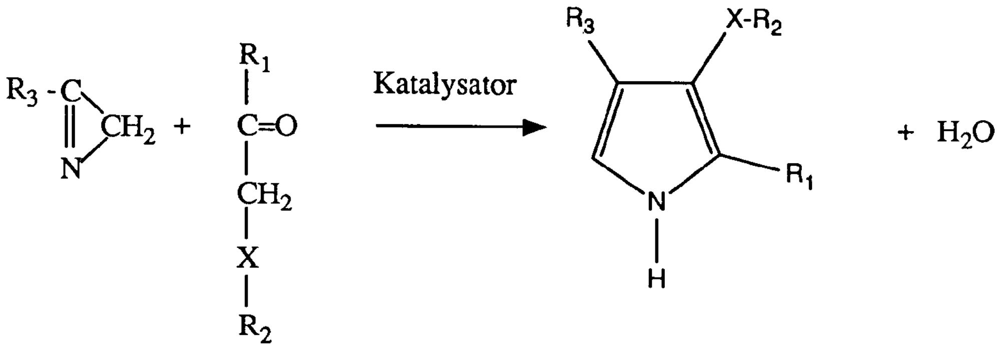


(IIa) (IIb) (I)

Die Umsetzung wird vorzugsweise in einem polaren, aprotischen organischen Lösungsmittel, wie z.B. Aceton, bei einer Temperatur von z.B. 10° bis 80°C, bevorzugt 30° bis 50°C, durchgeführt. Als Katalysator sind beispielsweise Übergangsmetallverbindungen, insbesondere Nickelacetylacetonat, geeignet.

Die Verbindungen der Formel I können bei der Herstellung auch als Gemisch anfallen. Dieses Gemisch enthält neben den Verbindungen der Formel I auch solche der Formel Ia,

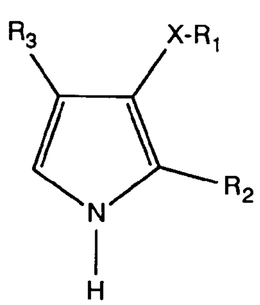

(Ia)

wobei das Molverhältnis von Verbindungen der Formel I zu Verbindungen der Formel Ia z.B. 1:4 bis 4:1 beträgt.

Dieses Gemisch kann, falls erforderlich, durch Kristallisation oder mit Hilfe von chromatographischen Methoden aufgetrennt werden.

Ebenfalls Gegenstand der Erfindung sind daher Zusammensetzungen enthaltend

a) ein chlorhaltiges Polymerisat und

b) ein Gemisch aus den Verbindungen der Formeln I und Ia,

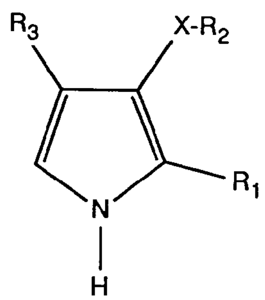

(I),

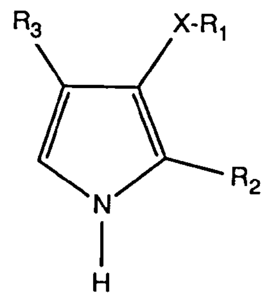

(Ia)

worin X, $R_1$, $R_2$ und $R_3$ die oben angegebenen Bedeutungen besitzen.

Die Verbindungen der Formeln IIa und IIb sind grösstenteils im Handel erhältlich, können aber auch in Analogie zu bekannten Verfahren (z.B.: S. Padmanabhan et al.; Bull. Chem. Soc. Jpn. <u>62</u>, 1358-1360 (1989); N. Kunieda et al.; Bull. Chem. Soc. Jpn. <u>54</u>, 1143 (1981)) hergestellt werden.

Die folgenden Beispiele erläutern die Erfindung weiter. Teil- und Prozentangaben beziehen sich darin, soweit nichts anderes angegeben ist, auf das Gewicht.

<u>Beispiel 1</u>: Herstellung von 2,4-Diphenyl-3-benzoylpyrrol.

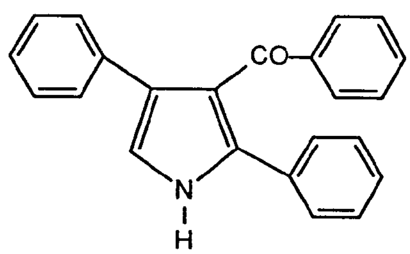

In eine Lösung von 5,6 g Dibenzoylmethan, 10 ml Aceton und 0,1 g Nickelacetylacetonat werden unter Rühren bei 50°C 3,0 g 2-Phenylazirin eingeaopft, das Gemisch anschliessend 30 Minuten am Rückfluss erwärmt, auf 20°C abgekühlt und mit 25 ml Wasser versetzt. Die ausgefallenen Kristalle werden abgesaugt, mit Wasser gewaschen und bis zur Gewichtskonstanz getrocknet.

Ausbeute: 7,8 g (≙ 96,3 % der Theorie) gelbe Kristalle.

Schmelzpunkt: 195- 196°C (Nach Umkristallisation aus Isopropanol).

Beispiele 2-6: Die in Tabelle 1 angegebenen Verbindungen werden in Analogie zu Beispiel 1 hergestellt.

Tabelle 1:

| Bsp. | Verbindung | Schmelzpunkt | Bemerkungen |
|---|---|---|---|
| 2 | 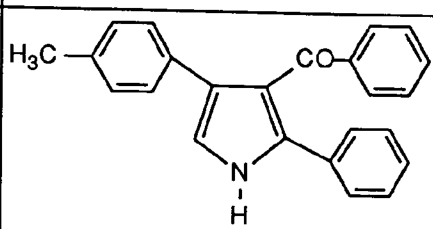 | 237°C | Als Ausgangsprodukt wird 2-(p-Methylphenyl)azirin eingesetzt. Das Reaktionsprodukt wird aus Essigester / Dimethylformamid umkristallisiert. |
| 3 | 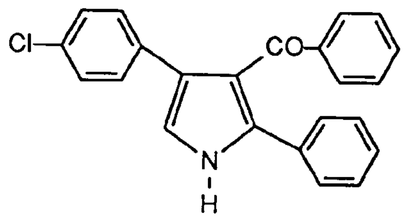 | 204-205°C | Als Ausgangsprodukt wird 2-(p-Chlorphenyl)azirin eingesetzt. Das Reaktionsprodukt wird aus Essigester umkristallisiert. |
| 4 | 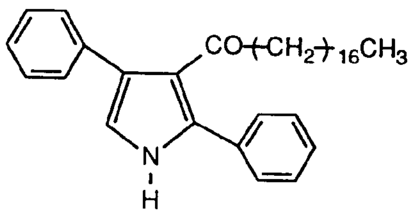 | 90-92°C | Die Verbindungen der Beispiele 4 und 5 fallen zusammen als Gemisch an und können durch Umkristallisation aus Petrolether getrennt werden. |
| 5 | 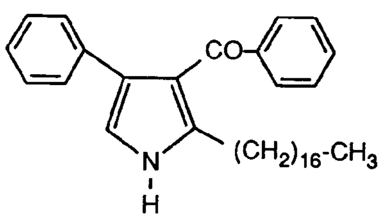 | $n_D^{30}$=1,5433 | |
| 6 | 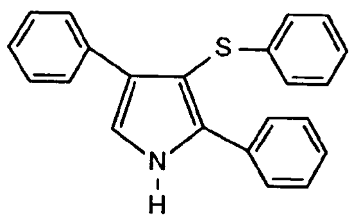 | 128-129°C | Das Reaktionsprodukt wird aus Isopropanol / $H_2O$ umkristallisiert. |

Beispiel 7: Herstellung eines Gemisches aus 2,4-Diphenyl-3-(3'-methylbutanoyl)pyrrol und 2-Isobutyl-3-benzoyl-4-phenylpyrrol im Molverhältnis 42:48.

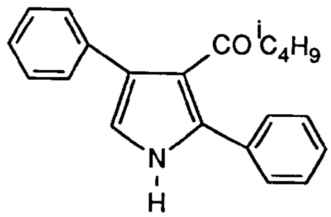

und

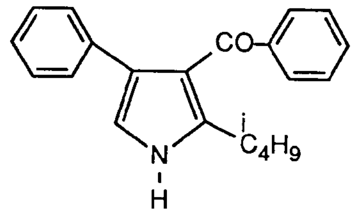

Die Herstellung erfolgt in Analogie zu Beispiel 1. Als Reaktanden werden 1-phenyl-heptan- 1,3-dion und 2-Phenylazirin eingesetzt. Nach Umkristallisation aus Petrol-ether/Toluol besitzt das Gemisch einen Schmelzpunkt von 118- 120°C.

Die beiden Komponenten des Gemisches können, falls gewünscht, mit Hilfe von chromatographischen Methoden aufgetrennt werden.

Beispiel 8: Herstellung eines Gemisches aus 2-(5′-Hydroxypentyl)-3-benzoyl-4-phenyl-pyrrol und 2,4-Diphenyl-3-(6′-hydroxyhexanoyl)pyrrol im Molverhältnis 1:1.

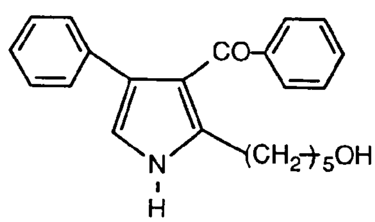

und

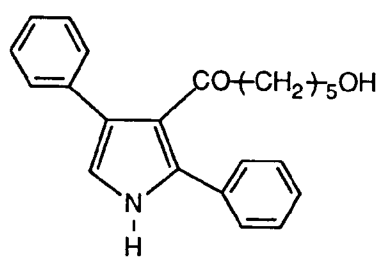

In eine Lösung von 30 ml Aceton, 23,4 g 1-Phenyl-8-hydroxyoctan-1,3-dion und 0,1 g Nickelacetylacetonat werden bei 25°C (Wasserbadkühlung) 11,7 g 2-Phenylazirin eingeaopft. Zur Vervollständigung der Reaktion wird das Gemisch noch 30 Minuten am Rückfluss erwärmt. Nach dem Abkühlen wird das Reaktionsgemisch in 150 ml Diethylether gelöst, mit Wasser und Natriumbicarbonat gewaschen, getrocknet und am Rotavapor auf Rückstand eingeengt.

Ausbeute: 20,5 g (= 61,6 % der Theorie) braunes Harz.

NMR-Spektrum:

Mehrere Multipletts mit insgesamt 10H: 1,0-3,6 ppm.
O-H: 2,0-2,3 ppm.
Zwei Dubletts (zusammen 1H): 6,6 und 6,7 ppm.
Multiplett, 10H: 6,9-7,7 ppm.

Die beiden komponenten des Gemisches können, falls gewünscht, mit Hilfe von chromatographischen Methoden aufgetrennt werden.

Beispiel 9: Herstellung eines Gemisches aus 2-Phenyl-3-octadecanoyl-4-p-chlorphenylpyrrol und 2-Heptadecyl-3-benzoyl-4-p-chlorphenylpyrrol im Molverhältnis 2: 3.

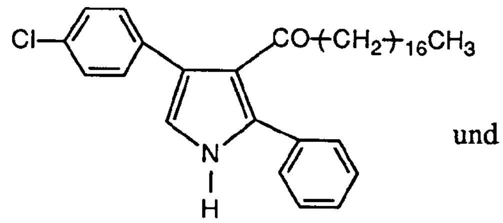

und

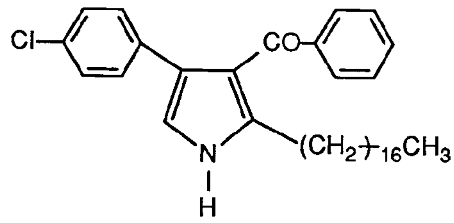

.

11,4 g p-Chlorphenylazirin werden bei 20°C in eine Lösung von 60 ml Aceton, 0,2 g Nickelacetylacetonat und 32,5 g 1-Phenyleicosan-1,3-dion eingeaopft und 30 Minuten am Rückfluss erwärmt. Am Rotavapor wird das Lösungsmittel abdestilliert und anschliessend der Rückstand aus 500 ml Acetoniail umkristallisiert. Die Kristalle werden abgesaugt und getrocknet.
Ausbeute: 33,0 g ($\hat{=}$ 85 % der Theorie) weisse Kristalle.
Schmelzpunkt: 58-76°C.

Die beiden komponenten des Gemisches können, falls gewünscht, mit Hilfe von chromatographischen Methoden aufgetrennt werden.

Beispiel 10: Herstellung von 2-Phenyl-3-octadecanoyl-4-p-chlorphenylpyrrol.

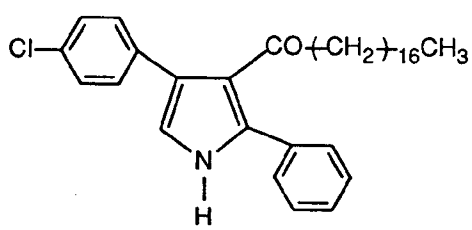

Die Mutterlauge, die bei der Herstellung gemäss Beispiel 9 anfällt, wird auf 0°C abgekühlt und die ausgefallenen Kristalle werden isoliert.
Ausbeute: 6,0 g ($\hat{=}$ 15 % der Theorie) weisse Kristalle.
Schmelzpunkt: 46-50°C.

Beispiel 11: Herstellung eines Gemisches aus 2-Phenyl-3-octadecanoyl-4-p-methylphenylpyrrol und 2-Heptadecyl-3-benzoyl-4-p-methylphenylpyrrol im Molverhältnis 45:55.

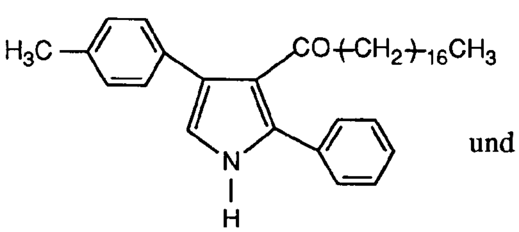

und

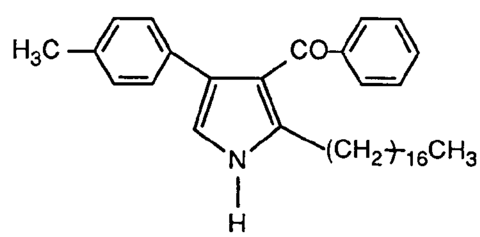

.

Die Herstellung erfolgt in Analogie zu Beispiel 9. Als Reaktanden werden 2-p-Methylphenylazirin und 1-Phenyleicosan-1,3-dion eingesetzt. Das erhaltene Produkt wird aus 300 ml Acetonitril umkristallisiert; die Kristalle werden isoliert und getrocknet.
Ausbeute: 22,3 g ($\hat{=}$ 60 % der Theorie) gelbe Kristalle.
Schmelzpunkt: 100- 113°C.

Die beiden komponenten des Gemisches können, falls gewünscht, mit Hilfe von chromatographischen Methoden aufgetrennt werden.

Beispiel 12: Herstellung von 2-Phenyl-3-octadecanoyl-4-p-methylphenylpyrrol.

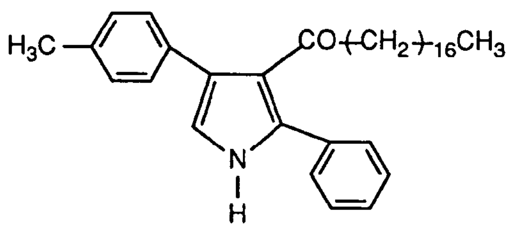

Die Mutterlauge, die bei der Herstellung gemäss Beispiel 11 anfällt, wird auf 0°C abgekühlt und die ausgefallenen Kristalle werden isoliert.
Ausbeute: 8,1 g (≙ 21,6 % der Theorie) hellgelbe Kristalle.
Schmelzpunkt: 46-48°C.

Beispiel 13: Herstellung eines Gemisches aus 2,4-Diphenyl-3-acetylpyrrol und 2-Methyl-3-benzoyl-4-phenylpyrrol im Molverhältnis 55:45.

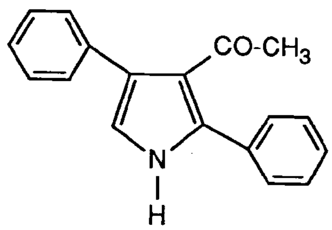

und

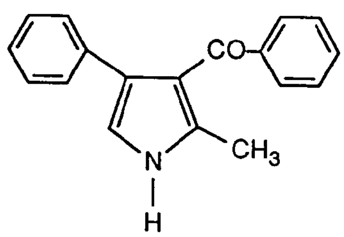

.

Die Herstellung erfolgt in Analogie zu Beispiel 1. Es werden 2-Phenylazirin und Benzoylaceton als Reaktanden eingesetzt.
Ausbeute: 97 % der Theorie.
Schmelzpunkt: 196-205 °C.

Die beiden Komponenten des Gemisches können, falls gewünscht, mit Hilfe von chromatographischen Methoden aufgetrennt werden.

Beispiel 14: Herstellung von 2-Ethyl-3-benzoyl-4-phenylpyrrol.

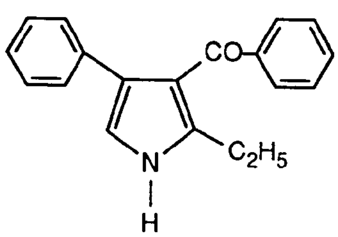

Die Herstellung erfolgt in Analogie zu Beispiel 1. Es werden 2-Phenylazirin und 1-Phenylpentan-1,3-dion

als Reaktanden eingesetzt. Das erhaltene Reaktionsprodukt wird in 150 ml heissem Ethanol gelöst und bei Raumtemperatur über Nacht stehengelassen. Die ausgefallenen Kristalle werden abgesaugt.
Schmelzpunkt: 175- 178°C.

Beispiel 15: Herstellung von 2,4-Diphenyl-3-propanoylpyrrol.

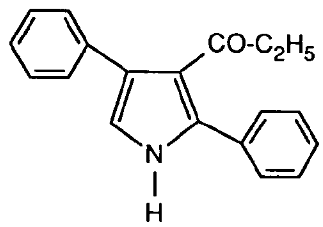

Die ethanolische Mutterlauge, die gemäss Beispiel 14 anfällt, wird abgekühlt und der erhaltene Niederschlag abgesaugt.
Schmelzpunkt: 135- 145°C.

Beispiel 16: Herstellung von 2-Phenyl-3-benzoyl-4-p-methoxyphenylpyrrol.

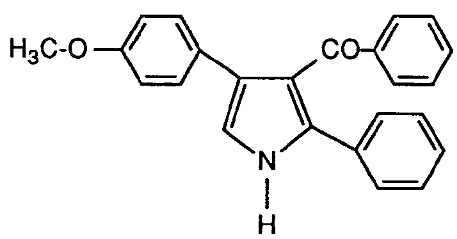

Die Herstellung erfolgt in Analogie zu Beispiel 1. Es werden Dibenzoylmethan und 2-(p-Methoxyphenyl)azirin eingesetzt.
Schmelzpunkt: 161-163°C.

Beispiel 17: Hitzetest.

Eine Trockenmischung bestehend aus 100 Teilen PVC (K-Wert 60), 5 Teilen epoxidiertem Sojabohnenöl, 0,2 Teilen Polyethylen-Wachs, 0,8 Teilen Phenyl-diisodecylphosphit und 1 Teil des in den Tabellen 2a bis 2c angegebenen Stabilisators wird auf einem Mischwalzwerk 5 Minuten bei 180°C gewalzt. Vom gebildeten 0,3 mm dicken Walzfell werden Folienmuster in einem Testofen (® Mathis-Thermotester Typ LFT-ST) bei 180°C thermisch nachbelastet und es wird die Zeit regisaiert, nach der die Probe zerfällt (Zerfallszeit).
Die Ergebnisse sind in den folgenden Tabellen 2a bis 2c zusammengefasst. Lange Zerfallszeiten bedeuten eine gute Stabilisierung des Polymeren.

Tabelle 2a:

| Stabilisator | Zerfallszeit in Minuten |
|---|---|
| Verbindung aus Beispiel 2 | 130 |
| Gemisch aus Beispiel 9 | 135 |

Tabelle 2b:

| Stabilisator | Zerfallszeit in Minuten |
|---|---|
| Verbindung aus Beispiel 1 | 135 |

Tabelle 2c:

| Stabilisator | Zerfallszeit in Minuten |
|---|---|
| Verbindung aus Beispiel 16 | >135 |

Beispiel 18: Belichtungstest.

Eine Trockenmischung bestehend aus 100 Teilen PVC (X-Wert 60), 5 Teilen epoxidiertem Sojabohnenöl, 0,2 Teilen Polyethylen-Wachs, 0,8 Teilen Phenyl-diisodecylphosphit, 0,02 Teilen Zn-Stearat und 1 Teil des in Beispiel 1 angegebenen Stabilisators wird auf einem Mischwalzwerk 5 Minuten bei 180°C gewalzt. Vom gebildeten 0,3 mm dicken Walzfell werden Folienmuster mit einer ®OSRAM ULTRA-VITALUX Lampe (300 Watt) im Abstand von 50 cm bestrahlt (durchschnittliche Belastungstemperatur: ca. 65°C). Dabei werden die Folienmuster in einem 24 Stunden Takt bestrahlt, bei dem 8 von 24 Stunden unter einer ca. 2 cm dicken Wasserschicht und die restlichen 16 Stunden trocken belichtet wird.
Im angegebenen Zeitintervall wird an einem Prüfmuster der "Yellowness Index [YI] " nach ASTM 1925 bestimmt.
Die Ergebnisse sind in der folgenden Tabelle 3 zusammengefasst.

Tabelle 3:

| Belastung in Stunden | 0 | 8 | 24 | 48 | 72 |
|---|---|---|---|---|---|
| YI | 16,4 | 30,1 | 54,1 | 69,9 | 96,7 |

Beispiel 19: Hitzetest.

Eine Trockenmischung bestehend aus 100 Teilen PVC, 5 Teilen epoxidiertem Sojabohnenöl, 0,35 Teilen Calciumstearat, 0, 15 Teilen Zinkstearat, 0,2 Teilen Phenyleicosan1,3-dion, 15 Teilen Dioctylphthalat, 0,1 Teilen 2-Hydroxy-4-methoxybenzophenon und 0,3 Teilen des in Tabelle 4 angegebenen Stabilisators wird auf einem Mischwalzwerk 5 Minuten bei 180°C gewalzt. Vom gebildeten 0,3 mm dicken Walzfell werden Folienmuster in einem Testofen (®Mathis-Thermotester Typ LFT-ST) bei 180°C thermisch nachbelastet und es wird die Zeit regisaiert, nach der die Probe zerfällt (Zerfallszeit).

Die Ergebnisse sind in der folgenden Tabelle 4 zusammengefasst. Lange Zerfallszeiten bedeuten eine gute Stabilisierung des Polymeren.

Tabelle 4:

| Stabilisator | Zerfallszeit in Minuten |
|---|---|
| Verbindung aus Beispiel 1 | >140 |
| Verbindung aus Beispiel 4 | 120 |

## Patentansprüche

1. Zusammensetzung enthaltend
   a) ein chlorhaltiges Polymerisat und
   b) mindestens eine Verbindung der Formel 1,

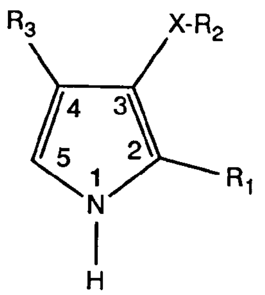

(I)

worin X eine Gruppe

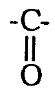 , -S- , 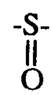

oder

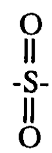

bedeutet, $R_1$ und $R_2$ unabhängig voneinander $C_1$-$C_{20}$-Alkyl, durch Hydroxy und/oder Halogen substituiertes $C_1$-$C_{10}$-Alkyl, $C_3$-$C_{20}$-Alkenyl, $C_5$-$C_{12}$-Cycloalkyl, durch $C_1$-$C_4$-Alkyl substituiertes $C_5$-$C_8$-Cycloalkyl, Phenyl oder durch 1 bis 3 Reste $A_1$ substituiertes Phenyl sind, wobei die Reste $A_1$ unabhängig voneinander $C_1$-$C_{10}$-Alkyl, Halogen, Hydroxy, Methoxy oder Ethoxy bedeuten,
$R_1$ und $R_2$ ferner $C_7$-$C_{10}$-Phenylalkyl oder am Phenyl durch 1 bis 3 Reste $A_2$ substituiertes $C_7$-$C_{10}$-Phenylalkyl sind, wobei die Reste $A_2$ unabhängig voneinander $C_1$-$C_{20}$-Alkyl, Halogen, Hydroxy, Methoxy oder Ethoxy bedeuten, und
$R_3$ Phenyl oder durch 1 bis 3 Reste $A_3$ substituiertes Phenyl ist, wobei die Reste $A_3$ unabhängig voneinander $C_1$-$C_{10}$-Alkyl, Halogen, Hydroxy, Methoxy, Ethoxy oder ($C_1$-$C_8$-Alkyl)oxycarbonyl sind, mit den Bedingungen, dass mindestens einer der Reste $R_1$ und $R_2$ Phenyl oder definitionsgemäss substituiertes Phenyl ist und $R_1$ verschieden von Methyl ist, wenn $R_2$ Phenyl bedeutet.

2. Zusammensetzung gemäss Anspruch 1, worin $R_1$ und $R_2$ unabhängig voneinander $C_1$-$C_{20}$-Alkyl, durch Hydroxy substituiertes $C_1$-$C_5$-Alkyl, $C_3$-$C_{17}$-Alkenyl, $C_5$-$C_7$-Cycloalkyl, durch $C_1$-$C_4$-Alkyl substituiertes $C_5$-$C_7$-Cycloalkyl, Phenyl, durch 1 bis 3 Reste $A_1$ substituiertes Phenyl, $C_7$-$C_{10}$-Phenylalkyl oder am Phenyl durch 1 bis 3 Reste $A_2$ substituiertes $C_7$-$C_{10}$-Phenylalkyl bedeuten und $R_3$ Phenyl oder durch 1 bis 3 Reste $A_3$ substituiertes Phenyl bedeutet.

3. Zusammensetzung gemäss Anspruch 1, worin $R_1$ und $R_2$ unabhängig voneinander $C_1$-$C_{17}$-Alkyl, durch Hydroxy substituiertes $C_1$-$C_5$-Alkyl, $C_3$-$C_{17}$-Alkenyl, Cyclohexyl, durch $C_1$-$C_4$-Alkyl substituiertes Cyclohexyl, Phenyl oder durch 1 bis 3 Reste $A_1$ substituiertes Phenyl sind, wobei die Reste $A_1$ unabhängig voneinander $C_1$-$C_4$-Alkyl, Chlor, Hydroxy, Methoxy oder Ethoxy bedeuten, $R_1$ und $R_2$ ferner Benzyl oder am Phenyl durch 1 bis 3 Reste $A_2$ substituiertes Benzyl sind, wobei die Reste $A_2$ unabhängig voneinander $C_9$-$C_{14}$-Alkyl, Chlor, Hydroxy, Metoxy oder Ethoxy bedeuten, und $R_3$ Phenyl oder durch 1 bis 3 Reste $A_3$ substituiertes Phenyl ist, wobei die Reste $A_3$ unabhängig voneinander $C_1$-$C_4$-Alkyl, Chlor, Hydroxy, Methoxy oder Ethoxy sind.

4. Zusammensetzung gemäss Anspruch 1, worin $R_1$ und $R_2$ unabhängig voneinander $C_1$-$C_{17}$-Alkyl, durch Hydroxy substituiertes $C_1$-$C_5$-Alkyl oder Phenyl bedeuten und $R_3$ Phenyl oder durch einen Rest $A_3$ substituiertes Phenyl ist, wobei $A_3$ $C_1$-$C_4$-Alkyl, Chlor oder Methoxy bedeutet.

5. Zusammensetzung gemäss Anspruch 1, worin $R_1$ Phenyl bedeutet.

6. Zusammensetzung gemäss Anspruch 1, worin $R_1$ und $R_3$ Phenyl bedeuten und $R_2$ Phenyl oder $C_4$-$C_{17}$-Alkyl ist.

7. Zusammensetzung gemäss Anspruch 1, worin X eine Gruppe

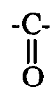

oder -S- ist.

8. Zusammensetzung gemäss Anspruch 1, enthaltend als komponente b) ein Gemisch aus den Verbindungen der Formeln I und Ia,

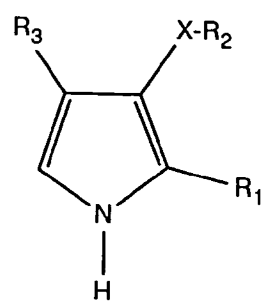 (I), 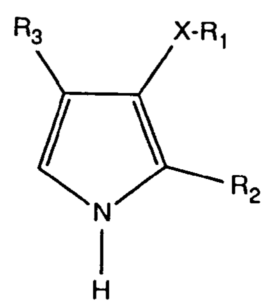 (Ia)

worin X, $R_1$, $R_2$ und $R_3$ die in Anspruch 1 angegebenen Bedeutungen besitzen.

9. Zusammensetzung gemäss Anspruch 1, worin das chlorhaltige Polymerisat Polyvinylchlorid ist.

10. Zusammensetzung gemäss Anspruch 1, enthaltend zusätzlich mindestens ein Me(II)-Carboxylat und/oder Me(II)-Phenolat, wobei Me(II) Ba, Ca, Mg, Cd oder Zn bedeutet.

11. Zusammensetzung gemäss Anspruch 1, enthaltend zusätzlich mindestens ein Me(II)-Carboxylat und/oder Me(II)-Phenolat, wobei Me(II) Ba, Ca, Mg, Cd oder Zn bedeutet, und/oder ein β-Diketon.

**12.** Zusammensetzung gemäss Anspruch 1, enthaltend zusätzlich eine Epoxyverbindung und/oder ein Phosphit.

**13.** Verwendung von Verbindungen der in Anspruch 1 definierten Formel I zum Stabilisieren eines chlorhaltigen Polymerisats gegen thermischen und lichtinduzierten Abbau.

**14.** Verbindungen der Formel Ib,

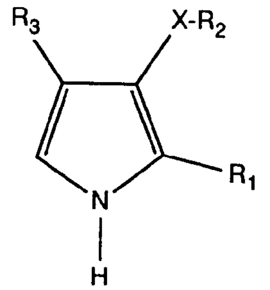

(Ib)

worin X eine Gruppe

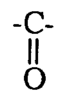 , 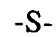 , 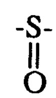

oder

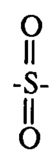

bedeutet, $R_1$ und $R_2$ unabhängig voneinander $C_1$-$C_{20}$-Alkyl, durch Halogen substituiertes $C_1$-$C_{10}$-Alkyl, $C_3$-$C_{20}$-Alkenyl, $C_5$-$C_{12}$-Cycloalkyl, durch $C_1$-$C_4$-Alkyl substituiertes $C_5$-$C_9$-Cycloalkyl, Phenyl oder durch 1 bis 3 Reste $A_1$ subsrituiertes Phenyl sind, wobei die Reste $A_1$ unabhängig voneinander $C_1$-$C_{10}$-Alkyl, Halogen, Hydroxy, Methoxy oder Ethoxy bedeuten,
$R_1$ und $R_2$ ferner $C_7$-$C_{10}$-Phenylalkyl oder am Phenyl durch 1 bis 3 Reste $A_2$ substituiertes $C_7$-$C_{10}$-Phenylalkyl sind, wobei die Reste $A_2$ unabhängig voneinander $C_1$-$C_{20}$-Alkyl, Halogen, Hydroxy, Methoxy oder Ethoxy bedeuten, und
$R_3$ Phenyl oder durch 1 bis 3 Reste $A_3$ substituiertes Phenyl bedeutet, wobei die Reste $A_3$ unabhängig voneinander $C_1$-$C_{10}$-Alkyl, Halogen, Hydroxy, Methoxy, Ethoxy oder ($C_1$-$C_8$-Alkyl)oxycarbonyl sind, mit den Bedingungen, dass mindestens einer der Reste $R_1$ und $R_2$ Phenyl oder definitionsgemäss substituiertes Phenyl ist und $R_1$ verschieden von Methyl und Phenyl ist, wenn $R_2$ Phenyl bedeutet.